# EUROPÄISCHE PATENTSCHRIFT

(11) **EP 2 764 169 B1**
(45) Veröffentlichungstag und Bekanntmachung des Hinweises auf die Patenterteilung: **17.06.2020**
(21) Anmeldenummer: 12783077.6
(22) Anmeldetag: 05.10.2012
(51) Int. Cl.: E03F 5/08, B05B 7/04, B01D 53/18, A61L 9/14

(54) **VERFAHREN UND EINE VORRICHTUNG FÜR DIE BEEINFLUSSUNG DES GERUCHES, WELCHER VON SCHACHTÖFFNUNGEN VON UNTERTAGE VERLAUFENDEN ABWASSERKANÄLEN AUSGEHT**
DEVICE AND METHOD FOR PREVENTING BAD ODOURS ARISING FROM MANHOLES OR INSPECTION CHAMBERS
DISPOSITIF ET METHODE POUR EMPECHER LES ODOURS DANS REGARD DE CHAUSSEE

(30) Priorität: 05.10.2011 DE 102011114956
(43) Veröffentlichungstag der Anmeldung: 13.08.2014
(73) Patentinhaber: Himmelfreundpointner, Kurt, 4612 Scharten (AT)
(72) Erfinder: Himmelfreundpointner, Kurt, 4612 Scharten (AT)
(86) Internationale Anmeldenummer: PCT/AT2012/000251
(87) Internationale Veröffentlichungsnummer: WO 2013/049867

(56) Entgegenhaltungen:
- EP-A1- 0 800 851
- WO-A1-2005/115553
- GB-A- 191 305 720

## Beschreibung

Die Erfindung betrifft ein Verfahren und eine Vorrichtung für die Beeinflussung des Geruches, welcher von Schachtöffnungen von untertage verlaufenden Abwasserkanälen ausgeht.

Durch sehr hoch - typischerweise auf Hausdächer - führende Kanalentlüftungsschächte wird zumeist ein Kamineffekt erreicht, durch welchen Luft aus dem untertage verlaufenden Kanal durch Rohre über die Dächer gefördert wird und durch die ebenerdig liegenden, vergitterten Kanalzugänge Luft eingesaugt wird. Wie bei Heizanlagen auch kann der Kamineffekt beispielsweise dann ausbleiben, wenn die Höhe zu gering ist und/oder, wenn bei sehr heißer Witterung die Umgebungsluft wärmer ist als die Luft im Kamin. Man kann sich helfen, indem man in oder an Kanalsystemen, bevorzugt wiederum in den nach oben führenden Entlüftungsrohren, Gebläse einbaut. Auf Grund der widrigen Umgebungsbedingungen sind die Gebläse in Anschaffung, Wartung und Betrieb relativ teuer.

Eine weitere gängige Methode ist es, in die Abwässer, vorwiegend schon in Auffangbehältern an größeren Einleitungsstellen, Mikroorganismen zu injizieren, welche eine Verringerung der Geruchsbildung des Abwassers bewirken. Die Schwächen der Methode liegen darin, dass es eine gewisse Zeit dauert, bis die erwünschte Wirkung der Mikroorganismen an einem eingeleiteten Abwasser eintritt und dass die Mikroorganismen bei bestimmten Zusammensetzungen des eingeleiteten Abwassers auch vorzeitig zerstört werden können.

In der JP 2002360682 wird vorgeschlagen, Wände von Abwasserrohren, Abwasserkanälen, und unterirdischen Auffangbehältern für Abwässer, sowie die Schachteinstiege zu derartigen Anlagen mit einem Pulver zur besprühen, welches Mikroorganismen enthält, welche in der Lage sind geruchsemittierende Substanzen aufzulösen, bzw. schon am Entstehen zu hindern. Schwächen hat die Methode vor allem dann, wenn die Geruchsbelastung vorwiegend von der im Kanal transportierten Flüssigkeit und nicht von den Wänden des Kanals kommt.

Die GB 191305720 A befasst sich schon 1913 mit der Zielsetzung, eine vergrößerte Menge von atmosphärischem Sauerstoff durch eine Schachtöffnungen eines untertage verlaufenden Abwasserkanals in den Abwasserkanal einzubringen und dazu Wasser, welches im Schacht versprüht wird, einzusetzen. Im oberen Schachtteil wird der Querschnitt des Schachtes durch einen Aufbau verengt, welcher etwa die Form der Mantelfläche eines Kegel- oder Pyramidenstumpfs hat. In die Verengung ist ein vertikal ausgerichtetes Stück Rundrohr eingesetzt, an dessen oberem Ende koaxial zum Rundrohr eine Sprüheinrichtung für Wasser angeordnet ist. Die Sprüheinrichtung umfasst eine zuführende Wasserleitung, eine feine Öffnung aus der das Wasser in einem Strahl vertikal nach unten herausspritzt, einen pyramidenförmige Prallkörper, auf dessen vertikal nach oben ausgerichtete Spitze der Strahl trifft und einen sich nach unten verengenden kegelstumpfmantelförmigen Ring, welcher am oberen Rand des Rundrohres aufliegt. Der aus der Leitung kommende Wasserstrahl wird durch den Prallkörper so aufgeweitet, dass er die Form eines Kegelmantels aufweist. Dieser Strahl trifft auf den kegelstumpfmantelförmigen Ring oder auf die Innenmantelfläche des Rundrohres und wird dort zerstäubt oder fließt daran vertikal nach unten. Optional kann unterhalb des Rundrohres ein Korb mit einem Granulat, das eine Art Deodorant enthalten kann, angeordnet sein, durch welchen das Wasser hindurch sickert und weiter in den Schacht hinunter tropft oder fließt. Im Schacht befindliche Luft kommt intensiv mit dem versprühten Wasser in Berührung, wird damit gekühlt und sinkt vermehrt nach unten. Von oben zuströmende Luft muss durch das erwähnte Rundrohr fließen und kommt damit zwangsweise stark mit dem versprühten Wasser in Kontakt.

Das nunmehr seit fast hundert Jahren bekannte Funktionsprinzip gemäß der GB 191305720 A wird bislang nicht eingesetzt und es wurde bislang auch nicht von der Fachwelt aufgegriffen, um in verbesserter Form angewendet zu werden. Vermutlich hat man sich durch die offensichtlichen Nachteile, dass ein hoher Wasserbedarf erforderlich zu sein scheint, dass die Kanalschächte durch die erforderlichen Vorrichtungen so sehr verengt werden, dass damit Wartungsarbeiten erschwert bis unmöglich werden, und dass im Winter Anwachsen von Eis auf den mit Wasser besprühten Anlagenteilen zu erwarten ist, abschrecken lassen.

Die EP 0800851 A1 beschreibt die Reinigung von schadstoffbelasteter Luft durch Zerstäubung von Wasser und dadurch Kondensieren und Binden von Schadstoffen. Das Zerstäuben des Wassers erfolgt unter Anwendung feiner Düsen, durch welche das Wasser hindurchgepresst wird durch Druckluft.

Die Luft wird durch einen motorbetriebenen Lüfter unabhängig von dem eingesprühten Wasser bewegt.

Die WO 2005/115553 A1 beschreibt das Reinigen von schadstoffbelasteter, durch eine Pumpe bewegter Luft in geschlossenen Räumen, indem in der Luft mit Ozon angereichertes Wasser unter Anwendung von Düsen und Druckluft versprüht wird, sodass in der Luft enthaltene Schadstoffe durch Oxidation in unschädliche Stoffe umgewandelt werden.

In den Schriften EP1369180 B1, DE19918120 A1, EP802827 B1, DE102004060535 A1, DE102005007805 A1, DE102006044439 A1, DE102007016481 A1, DE102007044272 A1 und DE102007055192 A1 sind Düsen für das Zerstäuben einer Flüssigkeit in sehr feine Tröpfchen (Durchmesser weniger als 0,1 mm) beschrieben. Diese Schriften sind nur ein kleiner beispielhafter Auszug aus einer sehr viel größeren Anzahl von Veröffentlichungen in denen derartige Düsen für das feinste zerstäuben von Flüssigkeiten beschrieben sind. Die Entwicklung der Technologie wurde stark für die Anwendung für das Einspritzen von Treibstoff beim Dieselmotor vorangetrieben.

Der Erfinder hat sich die Aufgabe gestellt eine verbesserte Methode zu schaffen, mit Hilfe derer Geruchsbelastung aus Abwasserkanalsystemen in der Umgebung von deren Öffnungen vermieden werden kann. Die zu schaffende Methode soll bei geringem Aufwand sicher und robust funktionieren.

Zum Lösen der Aufgabe wird von dem Prinzip entsprechend der GB 1913/5720 A ausgegangen, und die Aufgabe wird mit den Merkmalen der unabhängigen Ansprüche 1 und 4 gelöst.

Die Erfindung wird an Hand einer Zeichnung veranschaulicht:

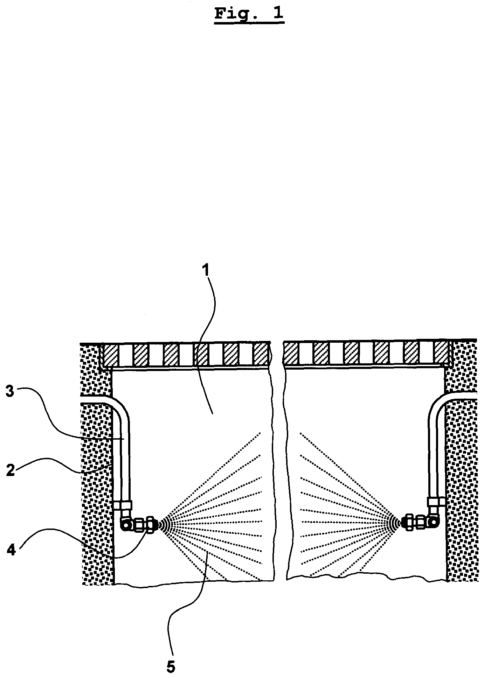

- Fig. 1: zeigt etwas stilisiert in seitlicher Teilschnittansicht den oberen Teil eines erfindungsgemäß ausgestatteten Kanalschachts.

Gemäß Fig. 1 sind am oberen Teil der Wände 2 eines Kanalschachts 1 Leitungen 3 befestigt, welche jeweils in einer Düse 4 enden. Die Leitungen 3 bilden das zuvor erwähnte, erfindungsgemäß erforderliche Druckgefäß. Wenn sie mit Wasser versorgt und unter Druck gesetzt sind, sprüht aus den Düsen 5 Wasser heraus und bildet einen Nebel 5 aus feinen Tröpfchen.

Durch die erfindungsgemäße Ausbildung der Düsen 4 mit feinen Düsenöffnungen ist die Größe der versprühten Tröpfchen so klein, dass die Tröpfchen bezogen auf ihr Volumen eine sehr große Oberfläche haben und somit sehr rasch verdampfen und damit erheblich Wärmeenergie aus der Umgebung absorbieren und somit die umgebende Luft stark kühlen, womit ein umgekehrter Kamineffekt erzielt wird - die Luft also im Kanalschacht 1 absinkt. Zusätzlich werden in der Luft enthaltene Moleküle und Ionen, welche Geruchsempfindung hervorrufen - des weiteren kurz "Geruchsstoffe" genannt - auch an Wassertröpfchen gebunden und damit zumindest teilweise neutralisiert. Gegenüber der vorbekannten Ausführung genügt für den gleichen Geruchsvermeidungseffekt etwa 1% der eingesprühten Wassermenge.

Idealerweise sind die Düsen möglichst nahe an der Wand 2 des Schachtes 1 angeordnet und bezüglich Ihrer Sprührichtung zur Querschnittsmitte des Schachtes 1 hin ausgerichtet.

Das aus den Düsen 4 strömende Wasser braucht nicht auf einen Prallkörper hin ausgerichtet zu sein um daran zu zerstäuben. Durch die erfindungsgemäße Ausbildung der Düsen 4 ist das Wasser schon zu feinen Tröpfchen zerstäubt. Man braucht dadurch nur sehr wenig Wasser und keinen Prallkörper. Indem kein Prallkörper verwendet wird, entfällt Platzbedarf dafür und es können auch keine Probleme durch Eisbildung am Prallkörper oder durch Erosion des Prallkörpers zufolge eines auftreffenden Wasserstrahls auftreten. Die Querschnittsfläche des Schachtes 1 braucht durch die bei der erfindungsgemäße Bauweise zu verwendenden Bauteile, nämlich Leitungen 3 und Düsen 4 kaum verengt zu werden, da diese Bauteile direkt an den Wänden 2 des Schachtes 1 angeordnet sein können.

Die Ausrichtung der Düsen 4 braucht nicht wie bei der vorbekannten Bauweise auf einen Prallkörper gerichtet zu sein. Damit entfällt Platzbedarf

Insgesamt ist damit gegenüber der aus der GB 1913/5720 A vorbekannten Ausführung ein viel einfacherer, ein viel besser raumsparender Aufbau und ein viel robusterer Aufbau möglich.

Um Vereisung von Düsen 4 und Leitungen 3 zuverlässig zu vermeiden, kann man an die Leitungen 3 und an die Düsen 4 einen Frostwächter in Form eines ohmschen elektrischen Leiters anbringen, welcher um die Leitungen und die Düsen 4 gewickelt ist und im Bedarfsfall durch elektrischen Stromfluss erwärmt wird. Derartige Frostwächter sind jetzt schon typischerweise als Dachrinnenheizung oder als Frostschutz für Trinkwasserversorgungsleitungen im Einsatz, weswegen hier nicht näher darauf eingegangen zu werden braucht. Man kann den Heizbedarf verringern, indem man Leitungen 3 und Düsen 4 (ausschließlich dem Öffnungsbereich der Düsen 4) mit einem wärmeisolierenden Material ummantelt.

Düsen 4, welche die genannten Anforderungen erfüllen sind ohne weiteres schon einige Jahrzehnte lang auf dem Markt erhältlich und beispielsweise entsprechend den in den eingangs erwähnten, Düsen betreffenden Dokumenten aufgebaut.

Im Allgemeinen ist die Wirkung der Düsen 4 besser, wenn die versprühten Tröpfchen möglichst klein sind. Beispielsweise werden Düsen angeboten, bei denen sich bei einem Wasserdruck im Druckbehältnis von 15 bar Tröpfchen mit einem durchschnittlichen Durchmesser von 32 µm versprühen lassen, wobei pro Stunde 1,8 bis 6 Liter Wasser versprüht werden.

Auf Grund verbreiteter Normierungen für Wasserinstallationsteile ist es vor allem aus wirtschaftlichen Gründen vorteilhaft einen Wasserdruck von nicht mehr als 6 bar vorzusehen. Man kann damit auch durchaus das Auslangen finden.

Bevorzugt ist dem eingesprühten Wasser mindestens eine Deodorant und/oder mindestens ein Parfum zugesetzt. Ein Deodorant in diesem Sinn ist ein chemischer oder mikrobiologischer Wirkstoff durch welchen Geruchsstoffe so umgewandelt werden, dass diese weniger oder gar nicht mehr störend riechen. Je nach Mittel kann dieses Umwandeln durch chemische Veränderung oder durch bakterielle Zersetzung erfolgen. Ein Parfum in diesem Sinn hingegen ist ein Stoff, welcher selbst vorteilhaft riecht. Durch beide derartige Wirkstoffe - Deodorants und Parfüme - wird die Wirkung des erfindungsgemäßen Verfahrens verstärkt.

Es kann vorteilhaft sein, das Aussprühen von Flüssigkeit mit dem Ausströmen von unter Druck stehender Luft zu kombinieren. Gegenüber dem Versprühen von Flüssigkeit allein kann man mit niedrigerem Druck ein feineres Versprühen der Flüssigkeit erreichen. Indem die Flüssigkeit in feinere Tröpfchen zersprüht wird, kann bei geringerem Flüssigkeitsverbrauch örtlich eine raschere Abkühlung durch Verdunstungskälte und auch durch die Kühlwirkung bei der Expansion von Druckluft erreicht werden. In manchen Fällen kann apparativer Aufwand verringert werden, da für den gleichen Kühleffekt Leitungen und Armaturen nicht für so hohe Drücke ausgelegt werden müssen.

Bevorzugt ist die Sprühvorrichtung mit einer mit Sensoren versehenen Steuerungsvorrichtung verbunden, durch welche sie in Abhängigkeit von Umgebungsbedingungen in der äußeren umgebenden Atmosphäre und in der Atmosphäre im Kanal ein- bzw. ausgeschaltet wird. Physikalische Größen die hier als "Umgebungsbedingung" gelten sind typischerweise Temperaturen, Strömungsrichtung und Strömungsgeschwindigkeit von Luft im Kanal sowie Konzentration von solchen Stoffen in der Umgebungsluft die als kennzeichnend für eine Geruchsbelastung gelten können. Das Herausfinden der optimalen Schaltzustände für jeden Schacht geschieht sicherlich überwiegend empirisch. Das heißt, dass vor allem mit hinzukommender Erfahrung klar wird, bei welchem Schacht bei welchem Witterungszustand welcher Schaltzustand optimal ist und welche gemessenen Konzentrationen an den jeweiligen Stellen entscheidend sind.

Bevorzugt schaltet die Steuerungsvorrichtung auch in Abhängigkeit von einem Zeitplan. Damit kann vorausschauend beispielsweise auf typische tageszeitliche Schwankungen von Witterungsverhältnissen, auf jahreszeitentypische Witterungsverhältnisse und auch auf mit dem Aktivitätsrhythmus von Menschen wechselnde Belastungen eingestellt werden.

Es ist zu beachten, dass die erfindungsgemäß hervorgerufenen Gasströmungen, welche durch Schächte in ein Kanalsystem hinein ausgerichtet sind, auf Grund von Verdrängung von Gas im Kanalsystem an einer oder mehreren Stellen auch Strömungen von Gasen aus dem Kanalsystem heraus hervorrufen. Bei der erfindungsgemäßen Ausstattung eines Kanalsystems sollte man also auch passend viele und ausreichend nahe Ausströmungsöffnungen für verdrängtes Gas aus dem Kanalsystem vorsehen und diese Öffnungen an Orte legen, an denen eine Geruchsbelastung möglichst nicht stört.

Zur Bedeutung des Begriffes "Schacht" in dieser Schrift sei hiermit klargestellt, dass damit nicht nur ein vertikaler, oben mit einem Deckel verschließbarer Einstieg in einen Kanal gemeint ist, sondern allgemein abfallende Einmündungen in ein Kanalsystem.

Die Erfindung ist natürlich besonders wertvoll an solchen Einmündungen anwendbar, welche man sich klassischerweise als "Schacht" vorstellt. Es treten aber auch Vorteile auf, wenn es sich um kleinere und nicht senkrecht abfallende Zuführungen in ein Kanalsystem handelt, wenn diese Zuführungen in ihrem oberen Längsbereich nicht vollständig gegen die Umgebungsluft abgedichtet sind.

## Patentansprüche

1. Verfahren für das Vermeiden von Geruchsbelastung aus einem Abwasserkanalsystem in der Umgebung eines zur Umgebungsluft hin offenen Schachtes (1) des Abwasserkanalsystems wobei in die Luft im Schacht (1) Wassertröpfchen versprüht werden, wobei das Wasser durch hydrostatischen Druck aus einem Druckgefäß (3) heraus in den Schacht (1) bewegt wird, wobei der Druck im Druckgefäß (3) mindestens 2 bar beträgt,
wobei das Wasser durch eine oder mehrere feine Düsen (4) aus dem Druckgefäß (3) herausbewegt wird wobei die Öffnungen der einen oder mehreren Düsen einen Durchmesser von weniger als 130 Mikrometern aufweisen.

2. Verfahren nach Anspruch 1, **dadurch gekennzeichnet, dass** der Sprühvorgang durch eine Steuerung eingestellt wird, welche die Einstellung des Sprühvorgangs in Abhängigkeit von Umgebungsbedingungen vornimmt, die über Sensoren festgestellt werden.

3. Verfahren nach Anspruch 1 oder 2, **dadurch gekennzeichnet, dass** der Sprühvorgang durch eine Steuerung eingestellt wird, welche die Einstellung des Sprühvorganges zumindest unter anderem auch in Abhängigkeit von einem Zeitplan vornimmt.

4. Abwasserkanalsystem mit einem zur Umgebungsluft hin offenen Schacht (1), wobei im Schacht (1) eine Sprühvorrichtung angeordnet ist, welche dazu in der Lage ist, in die Luft im Schacht Wassertröpfchen einzubringen, wobei die Sprühvorrichtung ein Wasser enthaltendes Druckgefäß (3) umfasst, in welchem ein hydrostatischer Druck herrscht, welcher gegenüber dem Umgebungsdruck um mindestens 2 bar erhöht ist, und wobei das Druckgefäß (3) eine oder mehrere feine Düsen (4) aufweist, durch welche hindurch Wasser aus dem Druckgefäß (3) entweichen kann, **dadurch gekennzeichnet, dass**
der Durchmesser der Öffnungen der einen oder mehreren Düsen (4) weniger als 130 µm beträgt.

5. Abwasserkanalsystem nach Anspruch 4, **dadurch gekennzeichnet, dass** eine Düse (4) an einer Wand (2) des Schachtes (1) positioniert ist und ihre Sprührichtung zur Mitte der Querschnittsfläche des Schachtes (1) hin ausgerichtet ist.

6. Abwasserkanalsystem nach Anspruch 4 oder 5, **dadurch gekennzeichnet, dass** das Druckgefäß (3) eine Wasserleitung ist.

7. Abwasserkanalsystem nach einem der Ansprüche 4 bis 6, **dadurch gekennzeichnet, dass** Druckgefäß (3) und Düse (4) eine wärmeisolierende Ummantelung aufweisen.

8. Abwasserkanalsystem nach einem der Ansprüche 4 bis 7, **dadurch gekennzeichnet, dass** Druckgefäß (3) und Düse (4) mit einer als Frostwächter wirkenden Heizvorrichtung versehen sind.

## Claims

1. Method for avoiding odour pollution from a sewer system in the vicinity of a shaft (1) of the sewer system which is open to the ambient air, whereby water droplets are sprayed into the air in the shaft (1), whereby the water is released through hydrostatic pressure from a pressure vessel (3) into the shaft (1), whereby the pressure in the pressure vessel (3) is at least 2 bar, whereby the water is moved out of the pressure vessel (3) through one or more fine nozzles (4), whereby the openings of the one or more nozzles have a diameter of less than 130 micrometres.

2. Method according to claim 1, **characterised in that** the spraying process is set by a control system which sets the spraying process depending on ambient conditions, which are determined by sensors.

3. Method according to claim 1 or 2, **characterised in that** the spraying process is set by a control system which sets the spraying process, including, inter alia, depending on a schedule.

4. Sewer system with a shaft (1) which is open to the ambient air, whereby a spray device is arranged in the shaft (1), which is capable of introducing water droplets into the air in the shaft, whereby the spray device comprises a pressure vessel (3) containing water which is under hydrostatic pressure which is at least 2 bar higher than the ambient pressure, and whereby the pressure vessel (3) has one or more fine nozzles (4) through which water can escape from the pressure vessel (3),
**characterised in that**
the diameter of the openings of the one or more nozzles (4) is less than 130 µm.

5. Sewer system according to claim 4, **characterised in that** a nozzle (4) is positioned on a wall (2) of the shaft (1) and its spray direction is oriented towards the centre of the cross-sectional area of the shaft (1).

6. Sewer system according to claim 4 or 5, **characterised in that** the pressure vessel (3) is a water pipe.

7. Sewer system according to claim 4 or 6, **characterised in that** the pressure vessel (3) and the nozzle (4) have a heat-insulated sheathing.

8. Sewer system according to claim 4 or 7, **characterised in that** the pressure vessel (3) and the nozzle (4) have a heating device acting as a frost guard.

## Revendications

1. Procédé pour éviter le dégagement d'odeurs depuis un système d'évacuation des eaux usées dans l'environnement d'un puits (1) ouvert vers l'air extérieur du système d'évacuation des eaux usées, dans lequel des gouttelettes d'eau sont pulvérisées dans l'air dans le puits (1), l'eau étant extraite d'un récipient sous pression (3) dans le puits (1) par pression hydrostatique, la pression dans le récipient sous pression (3) étant au moins de 2 bar, l'eau étant transportée en-dehors du récipient sous pression (3) par une ou plusieurs buses fines (4) et les ouvertures de la ou des buses présentant un diamètre inférieur à 130 micromètres.

2. Procédé selon la revendication 1, **caractérisé en ce que** le processus de pulvérisation est réglé par une commande qui procède au réglage du processus de pulvérisation en fonction des conditions ambiantes qui sont recueillies par des capteurs.

3. Procédé selon la revendication 1 ou 2, **caractérisé en ce que** le processus de pulvérisation est réglé par une commande qui procède au réglage du processus de pulvérisation au moins aussi en fonction d'un programme.

4. Système d'évacuation des eaux usées avec un puits (1) ouvert vers l'air extérieur, dans lequel il est agencé dans le puits (1) un dispositif de pulvérisation qui est capable d'introduire des gouttelettes d'eau dans l'air dans le puits, le dispositif de pulvérisation comprenant un récipient sous pression (3) contenant de l'eau dans lequel règne une pression hydrostatique, laquelle pression hydrostatique étant augmentée d'au moins 2 bar par rapport à la pression ambiante, et le récipient sous pression (3) présentant une ou plusieurs buses fines (4) permettant de laisser échapper de l'eau du récipient sous pression (3),
**caractérisé en ce que**
le diamètre des ouvertures de la ou des buses (4) est inférieur à 130 pm.

5. Système d'évacuation des eaux usées selon la revendication 4, **caractérisé en ce qu'**une buse (4) est positionnée sur une paroi (2) du puits (1) et **en ce que** la direction de projection est orientée vers le milieu de la section transversale du puits (1).

6. Système d'évacuation des eaux usées selon la revendication 4 ou 5, **caractérisé en ce que** le récipient sous pression (3) est une canalisation d'eau.

7. Système d'évacuation des eaux usées selon l'une des revendications 4 à 6, **caractérisé en ce que** le récipient sous pression (3) et la buse (4) présentent une enveloppe thermiquement isolante.

8. Système d'évacuation des eaux usées selon l'une des revendications 4 à 7, **caractérisé en ce que** le récipient sous pression (3) et la buse (4) sont munis d'un dispositif de chauffage agissant comme un antigel.
